# EUROPEAN PATENT APPLICATION

(11) **EP 4 548 940 A1**
(43) Date of publication of application: **07.05.2025**
(21) Application number: 24156223.0
(22) Date of filing: 07.02.2024
(51) Int. Cl.: A61M 1/00, A47L 9/24, A47L 9/32

(54) **RETRACTABLE SUCTION**

(30) Priority: 31.10.2023 CN 202322939157 U
(71) Applicant: Inc (Shanghai) Hospital Management Co., Ltd., Shanghai 201204 (CN)
(72) Inventor: SHEN, Shanghang, Shanghai, 201204 (CN)
(74) Representative: Zaboliene, Reda

(57) **Abstract**

Disclosed is a retractable suction. The retractable suction includes a handle sleeve, a fastening sleeve and a linear aspiration tube; the inside of the handle sleeve sequentially includes a first straight tube section and a conical section along its axis; the inner diameter of the end, close to the first straight tube section, of the conical section is the minimum, and the minimum inner diameter of the conical section is larger than or equal to the inner diameter of the first straight tube section; the fastening sleeve is sleeved on the linear aspiration tube; one end of the linear aspiration tube is inserted into the handle sleeve, a fitting surface is disposed on the outer side of the fastening sleeve, the minimum diameter of the fitting surface is larger than the minimum inner diameter of the conical section.

## Description

### CROSS-REFERENCE TO RELATED APPLICATIONS

This application claims priority to Chinese Patent Application No. 202322939157.8, filed on October 31, 2023, which is incorporated herein by reference in its entirety.

### TECHNICAL FIELD

The application relates to the technical field of medical instruments, and in particular to a retractable suction.

### BACKGROUND

Craniocerebral surgery has been a high-risk area of surgical operations all the time, which, with the continuous development of medical technology and medical instruments, has developed from initial craniotomy to various basis cranial operation through endoscopic observation through nasal cavity. The whole process of the basis cranial operation through the nasal cavity is carried out under the supervision of an endoscope, compared with craniotomy, it has the advantages of clearer operation process, small wound surface, convenient operation path and no trauma to the head and face, the occurrence of complications and the injury to patients are reduced to the maximum extent, and therefore, the basis cranial operation through the nasal cavity are increasingly popular with doctors and patients.

However, in the craniocerebral surgery through the nasal cavity, a transnasal suction becomes a very important surgical instrument needed in the operation, which is mainly used for sucking wound bleeding, washing liquid and intracranial special tissues (tumor, necrosis or cyst) to remove hematoma and keep a clear operation visual field. However, in the operation process of the craniocerebral surgery through the nasal cavity, the basis crani operation through the nasal cavity is deeper in operation position, narrower in space, and more precise in operation action, while the existing suction has fixed length and cannot extend or retract, so as to be only used for the focus at a specific position, and the adjustment requirement of a plurality of operations cannot be met. Therefore, the above disadvantages of the existing suction need to be improved to better meet the requirements of the craniocerebral surgery through the nasal cavity.

### SUMMARY

In order to overcome the defects of the relevant art, the application provides a retractable suction.

The application provides the following technical solution: a retractable suction includes a handle sleeve, a fastening sleeve and a linear aspiration tube. The handle sleeve is internally provided with an aspiration channel penetrating through its two ends, and the aspiration channel sequentially includes a first straight tube section and a conical section along its axis. The inner diameter of the end, close to the first straight tube section, of the conical section is the minimum, and the minimum inner diameter of the conical section is larger than or equal to the inner diameter of the first straight tube section. The fastening sleeve is sleeved on the linear aspiration tube and may slide on the linear aspiration tube. One end of the linear aspiration tube is inserted into the handle sleeve, a fitting surface matched with the inner wall surface of the conical section is disposed on the outer side of the end, close to the handle sleeve, of the fastening sleeve, the minimum diameter of the fitting surface is larger than the minimum inner diameter of the conical section, and at least one wire slot is formed in the fitting surface. The wire slot runs through the inner side and the outer side of the fastening sleeve, with one end, close to the first straight tube section, being open. When the end, close to the first straight tube section, of the wire slot elastically deforms to the smallest width, the linear aspiration tube is fixedly connected with the handle sleeve through the fastening sleeve, in such a case, a gap between the linear aspiration tube and the handle sleeve is sealed by the fastening sleeve.

Furthermore, in the application, a limiting ring is included, which is located in the first straight tube section and is fixedly connected with the linear aspiration tube.

In the application, furthermore, in the aspiration channel, the side, away from the conical section, of the first straight tube section is a second straight tube section, and the inner diameter of the second straight tube section is smaller than the outer diameter of the limiting ring.

Furthermore, in the application, the fastening sleeve sequentially includes a fastening section and a handle section along its axial direction. The fitting surface and the wire slot are formed on the fastening section, and the handle section is always located outside the handle sleeve.

Furthermore, in the application, a sealing section is further disposed between the fastening section and the handle section. In the aspiration channel, the side, away from the first straight tube section, of the conical section is a third straight tube section. The inner diameter of the third straight tube section is larger than or equal to the maximal inner diameter of the conical section, and the outer side surface of the sealing section is matched with the inner side surface of the third straight tube section.

Furthermore, in the application, decorative patterns are disposed on both the outer side surface of the handle sleeve and the outer side surface of the handle section.

Furthermore, in the application, a pagoda connector is included, which sleeves and is fixed at one end, away from the fastening sleeve, of the handle sleeve.

Furthermore, in the application, an aspiration connector is further included, the aspiration connector is fixed at the end, away from the handle sleeve, of the linear aspiration tube, and is internally provided with a resistance increasing structure, and the resistance increasing structure is of a concave-convex structure, a concave structure or a convex structure.

Furthermore, in the application, a plurality of resistance increasing structures are disposed in the aspiration connector, and all the resistance increasing structures are distributed along the axis of the aspiration connector.

Furthermore, in the application, the outer side surface of the aspiration connector is a conical surface, and the end, away from the handle sleeve, of the conical surface is a large diameter section; and the inner side surface of the aspiration connector is a cylindrical surface.

In summary, compared with the relevant art, the application has the following beneficial effects.

In the application, the linear aspiration tube is inserted into the fastening sleeve, the fastening sleeve is inserted into the handle sleeve, by virtue of elastic deformation of the fastening sleeve and friction force among the three, fixed connection among the three is completed, before fixed connection, the position of the fastening sleeve on the linear aspiration tube is adjusted, and then the length of the suction may be adjusted to meet adjustment requirement of a plurality of operations.

### BRIEF DESCRIPTION OF THE DRAWINGS

Fig. 1 is a schematic structure diagram of an embodiment.
Fig. 2 is a schematic structure diagram of a cross-sectional view of an embodiment.
Fig. 3 is a schematic structure diagram of a handle sleeve.
Fig. 4 is a schematic structure diagram of a fastening sleeve.

### DETAILED DESCRIPTION OF THE EMBODIMENTS

The technical solutions of the application will be clearly described below with reference to the accompanying drawings, apparently, the described embodiments are not all embodiments of the application, and all other embodiments obtained by those of ordinary skill in the art without creative efforts shall fall within the protection scope of the application.

It is to be noted that orientation or position relationships indicated by terms "center", "upper", "lower", "level", "left", "right", "front", "back", "horizontal", "longitudinal" and the like are orientation or position relationships shown in the drawings, are adopted not to indicate or imply that indicated apparatuses or components must be in specific orientations or structured and operated in specific orientations but only to conveniently and simply describe the present application and thus should not be understood as limits to the present application.

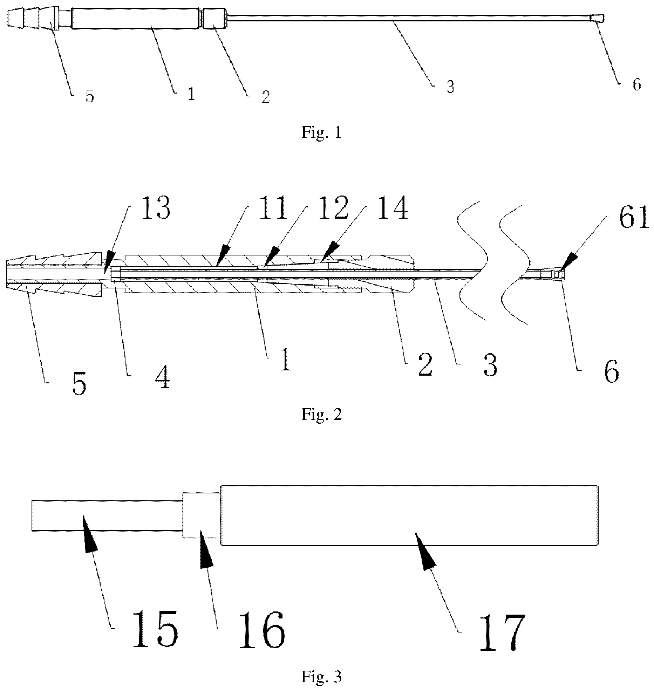

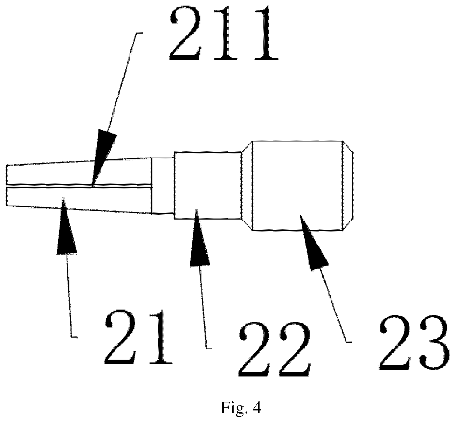

As shown in Figs. 1-4, the application provides a retractable suction, which includes a handle sleeve 1, a fastening sleeve 2 and a linear aspiration tube 3. The handle sleeve 1 is internally provided with an aspiration channel penetrating through its two ends, and the aspiration channel sequentially includes a first straight tube section 11 and a conical section 12 along its axis. The inner diameter of the end, close to the first straight tube section 11, of the conical section 12 is the minimum, the minimum inner diameter of the conical section 12 is larger than or equal to the inner diameter of the first straight tube section 11, and in the embodiment, the minimum inner diameter of the conical section 12 is larger than the inner diameter of the first straight tube section 11. The fastening sleeve 2 is sleeved on the linear aspiration tube 3 and may slide on the linear aspiration tube 3. One end of the linear aspiration tube 3 is inserted into the handle sleeve 1, a fitting surface matched with the inner wall surface of the conical section 12 is disposed on the outer side of the end, close to the handle sleeve 1, of the fastening sleeve 2, the minimum diameter of the fitting surface is larger than the minimum inner diameter of the conical section 12, and at least one wire slot 211 is formed in the fitting surface. The wire slot 211 runs through the inner side and the outer side of the fastening sleeve 2, with one end, close to the first straight tube section 11, being open.

As the contact area between the fitting surface and the conical section 12 increases, the fastening sleeve 2 is squeezed by the conical section 12 to have elastic deformation, the end, close to the first straight tube section 11, of the fastening sleeve 2 gradually shrinks, when the end, close to the first straight tube section 11, of the wire slot 211 elastically deforms to the smallest width, the linear aspiration tube 3 is fixedly connected with the handle sleeve 1 through the fastening sleeve 2, in such a case, a gap between the linear aspiration tube 3 and the handle sleeve 1 is sealed by the fastening sleeve 2.

Then, a vacuum negative pressure apparatus is connected with the end, away from the fastening sleeve 2, of the handle sleeve 1, and wound bleeding, exudate, pus, contents of organs and flushing fluid are sucked out through the end, located outside the handle sleeve 1, of the linear aspiration tube 3 to maintain a clear surgical field of view. Before fixed connection of the linear aspiration tube 3 with the handle sleeve 1 through the fastening sleeve 2, the position of the fastening sleeve 2 on the linear aspiration tube 3 is adjusted, and then the length of the suction may be adjusted to meet adjustment requirement of a plurality of operations.

Specifically, a plurality of wire slots 211 are available, and all the wire slots 211 are evenly distributed around the axis of the fastening sleeve 2 to ensure that the fastening sleeve 2 evenly applies clamping force around the axis of the linear aspiration tube 3. In the embodiment, four wire slots 211 are formed.

In the embodiment, a limiting ring 4 is further included, the limiting ring 4 is located in the first straight tube section 11 and is fixedly connected with the linear aspiration tube 3, and the disposing of the limiting ring 4 limits the maximum extending-retracting length of the suction and reduces the possibility that the linear aspiration tube 3 is separated from the fastening sleeve 2 in the extending-retracting process of the suction.

Specifically, the outer diameter of the limiting ring 4 is equal to the inner diameter of the first straight tube section 11. The fitting of the limiting ring 4 with the first straight tube section 11 may assist the fastening sleeve 2 in limiting the axis position of the linear aspiration tube 3, so as to complete fixing of the handle sleeve 1, the fastening sleeve 2 and the linear aspiration tube 3 more quickly.

Specifically, in the aspiration channel, the side, away from the conical section 12, of the first straight tube section 11 is a second straight tube section 13, and the inner diameter of the second straight tube section 13 is smaller than the outer diameter of the limiting ring 4. The fitting of the second straight tube section 13 and the limiting ring 4 limits the minimum extending-retracting length of the suction.

In the embodiment, the limiting ring 4 is fixed at the end of the linear aspiration tube 3, so as to effectively avoid impact of the linear aspiration tube 3 and the second straight tube section 13, and protect the linear aspiration tube 3. The fixing of the limiting ring 4 and the linear aspiration tube 3 may adopt various fixed connecting modes including welding, gluing, clamping, detachable fixed connection and the like, and welding is adopted in the embodiment.

Specifically, the fastening sleeve 2 sequentially includes a fastening section 21 and a handle section 23 along its axial direction. The fitting surface and the wire slot 211 are formed on the fastening section 21, and the handle section 23 is always located outside the handle sleeve 1. When the length of the suction is adjusted, it is not necessary to apply force to the fastening sleeve 2 through the linear aspiration tube 3, but directly to the fastening sleeve 2, and the fastening sleeve 2 is moved out of the handle sleeve 1, thus effectively protecting the linear aspiration tube 3.

Specifically, a sealing section 22 is further disposed between the fastening section 21 and the handle section 23. In the aspiration channel, the side, away from the first straight tube section 11, of the conical section 12 is a third straight tube section 14. The inner diameter of the third straight tube section 14 is larger than or equal to the maximal inner diameter of the conical section 12, and the outer side surface of the sealing section 22 is matched with the inner side surface of the third straight tube section 14. In the embodiment, the inner diameter of the third straight tube section 14 is larger than the maximal inner diameter of the conical section 12. Due to the disposing of the sealing section 22, on one hand, it is ensured that a negative pressure environment is formed inside the handle sleeve 1 to suck wound bleeding, exudate, pus, contents of organs and flushing fluid, and on the other hand, it may limit collinear axis of the fastening sleeve 2 and the handle sleeve 1, so that the time required for extending-retracting adjustment of the suction is further shortened.

Specifically, in the embodiment, the outer side face of the sealing section 22 is matched with the inner side face of the third straight tube section 14 by threads, the fastening sleeve 2 is connected with the handle sleeve 1 by threads, and by rotating the fastening sleeve 2, the fastening section 21 and the conical section 12 are squeezed. In the embodiment, external threads are disposed on the outer side surface of the sealing section 22, and internal threads are disposed on the inner side surface of the third straight tube section 14.

Specifically, decorative patterns are disposed on both the outer side surface of the handle sleeve 1 and the outer side surface of the handle section 23, so as to increase friction force between hands and the handle sleeve 1 and the fastening sleeve 2, and accelerate adjustment of relative position of the fastening sleeve 2 and the hand sleeve 1. The decorative patterns may be processed by knurling, surface drawing, surface sandblasting, surface corrosion, etc., and in the embodiment, the decorative patterns are processed by knurling. In the embodiment, beveling treatment is carried out on edges of two ends of the handle section 23.

In the embodiment, a pagoda connector 5 is further included, the pagoda connector 5 sleeves and is fixed at one end, away from the fastening sleeve 2, of the handle sleeve 1, so as to increase fastness of connection of the handle sleeve 1 and the vacuum negative pressure apparatus.

Specifically, the outer cylindrical surface of the handle sleeve 1 includes a first section 15, a second section 16 and a third section 17 along the axis of the handle sleeve 1, the diameter of the first section 15 is smaller than that of the second section 16, and the diameter of the second section 16 is smaller than that of the third section 17; and the first section 15 is inserted into the pagoda connector 5, the second section 16 limits the pagoda connector 5, and decorative patterns are disposed on the third section 17. In the embodiment, beveling treatment is carried out on edges of two ends of the third section 17.

Specifically, in the embodiment, the end, away from the second section 16, of the first section 15 is subjected to a tube expanding process for increasing the outer diameter, so that part of the outer diameter processed by the tube expanding process is larger than the inner diameter of the pagoda connector 5, and the pagoda connector 5 is prevented from separating from the handle sleeve 1. In the embodiment, the tube expanding process adopts riveting tube expanding.

In the embodiment, an aspiration connector 6 is further included, the aspiration connector 6 is fixed at the end, away from the handle sleeve 1, of the linear aspiration tube 3, and is internally provided with a resistance increasing structure 61, and the resistance increasing structure 61 is of a concave-convex structure, a concave structure or a convex structure. The resistance increasing structure 61 may change the original plane shape of the inner side surface of the aspiration connector 6, introduce additional shape resistance, reduce fluid speed, disturb fluid direction, increase momentum loss to be overcome by reverse flow of fluid, and effectively reduce the possibility of reverse flow. In the embodiment, the resistance increasing structure 61 is of a concave structure, and the concave structure is annular. The fixing of the aspiration connector 6 and the linear aspiration tube 3 may adopt various fixed connecting modes including welding, gluing, clamping, detachable fixed connection and the like, and welding is adopted in the embodiment.

Specifically, a plurality of resistance increasing structures 61 are disposed in the aspiration connector 6, and all the resistance increasing structures 61 are distributed along the axis of the aspiration connector 6, for further reducing the possibility of reverse flow of liquid in the aspiration connector. In the embodiment, two resistance increasing structures 61 are disposed.

Specifically, the inner side surface of the aspiration connector 6 is a cylindrical surface; the outer side surface of the aspiration connector 6 is a conical surface, the end, away from the handle sleeve 1, of the conical surface is a large-diameter end, so that the wall thickness of a fluid inlet end of the aspiration connector 6 is increased, the strength and pressure resistance of the fluid inlet end of the aspiration connector 6 are improved, and the service life of the aspiration connector 6 is prolonged.

In conclusion, in the embodiment, the linear aspiration tube 3 is inserted into the fastening sleeve 2, the fastening sleeve 2 is inserted into the handle sleeve 1, by virtue of elastic deformation of the fastening sleeve 2 and friction force among the three, fixed connection among the three is completed, before fixed connection, the position of the fastening sleeve 2 on the linear aspiration tube 3 is adjusted, and then the length of the suction may be adjusted to meet adjustment requirement of a plurality of operations.

The above is only preferred embodiments of the application and is not intended to limit the application, and those skilled in the art may make various modifications and variations. Any modifications, equivalent replacements, improvements and the like made within the spirit and principle of the application shall fall within the scope of protection of the application.

## Claims

1. A retractable suction, comprising a handle sleeve, a fastening sleeve and a linear aspiration tube; wherein
the handle sleeve is internally provided with an aspiration channel penetrating through two ends of the handle sleeve, and the aspiration channel comprises a first straight tube section and a conical section, the first straight tube section and the conical section are sequentially disposed along an axis direction;
an inner diameter of the end, close to the first straight tube section, of the conical section is the minimum, and a minimum inner diameter of the conical section is larger than or equal to an inner diameter of the first straight tube section;
the fastening sleeve is sleeved on the linear aspiration tube, and the fastening sleeve slides on the linear aspiration tube;
one end of the linear aspiration tube is inserted into the handle sleeve, a fitting surface matched with an inner wall surface of the conical section is disposed on an outer side of the end, close to the handle sleeve, of the fastening sleeve, a minimum diameter of the fitting surface is larger than the minimum inner diameter of the conical section, and at least one wire slot is formed in the fitting surface; and
the wire slot runs through the inner side and an outer side of the fastening sleeve, and the end, close to the first straight tube section, of the wire slot is open; when the end, close to the first straight tube section, of the wire slot elastically deforms to the smallest width, the linear aspiration tube is fixedly connected with the handle sleeve through the fastening sleeve, in such a case, a gap between the linear aspiration tube and the handle sleeve is sealed by the fastening sleeve.

2. The retractable suction according to claim 1, further comprising a limiting ring, wherein the limiting ring is located in the first straight tube section, and the limiting ring is fixedly connected with the linear aspiration tube.

3. The retractable suction according to claim 2, wherein in the aspiration channel, the side, away from the conical section, of the first straight tube section is a second straight tube section, and an inner diameter of the second straight tube section is smaller than the outer diameter of the limiting ring.

4. The retractable suction according to claim 1, wherein the fastening sleeve comprises a fastening section and a handle section, the fastening section and the handle section are sequentially disposed along an axial direction; the fitting surface and the wire slot are formed on the fastening section, and the handle section is always located outside the handle sleeve.

5. The retractable suction according to claim 4, wherein a sealing section is further disposed between the fastening section and the handle section; in the aspiration channel, the side, away from the first straight tube section, of the conical section is a third straight tube section; and an inner diameter of the third straight tube section is greater than or equal to a maximum inner diameter of the conical section, and an outer side surface of the sealing section is matched with an inner side surface of the third straight tube section.

6. The retractable suction according to claim 4, wherein decorative patterns are disposed on both outer side surface of the handle sleeve and outer side surface of the handle section.

7. The retractable suction according to claim 1, further comprising a pagoda connector, wherein the pagoda connector sleeves and is fixed at one end, away from the fastening sleeve, of the handle sleeve.

8. The retractable suction according to claim 1, further comprising an aspiration connector, wherein the aspiration connector is fixed at the end, away from the handle sleeve, of the linear aspiration tube, the aspiration connector is internally provided with a resistance increasing structure, and the resistance increasing structure is of a concave-convex structure, a concave structure or a convex structure.

9. The retractable suction according to claim 8, wherein a plurality of resistance increasing structures are disposed in the aspiration connector, and all the resistance increasing structures are distributed along an axis of the aspiration connector.

10. The retractable suction according to claim 9, wherein an outer side surface of the aspiration connector is a conical surface, and the end, away from the handle sleeve, of the conical surface is a large diameter section; and an inner side surface of the aspiration connector is a cylindrical surface.
